# EUROPÄISCHE PATENTSCHRIFT

(11) **EP 1 406 577 B1**
(45) Veröffentlichungstag und Bekanntmachung des Hinweises auf die Patenterteilung: **10.10.2007**
(21) Anmeldenummer: 02748757.8
(22) Anmeldetag: 11.06.2002
(51) Int. Cl.: A61Q 5/10, C07C 217/84

(54) **KUPPLERKOMPONENTEN FUR OXIDATIONSHAARFARBEN**
NOVEL COUPLER COMPONENTS FOR OXIDATIVE HAIR COLOURING
NOUVEAUX COMPOSANTS DE COPULATION DESTINES A LA COLORATION PAR OXYDATION DES CHEVEUX

(30) Priorität: 12.06.2001 DE 10128472
(43) Veröffentlichungstag der Anmeldung: 14.04.2004
(73) Patentinhaber: Henkel Kommanditgesellschaft auf Aktien, 40589 Düsseldorf (DE)
(72) Erfinder: ROSE, David, 40723 Hilden (DE); HÖFFKES, Horst, 40595 Düsseldorf (DE); MEINIGKE, Bernd, 51381 Leverkusen (DE)
(86) Internationale Anmeldenummer: PCT/EP2002/006352
(87) Internationale Veröffentlichungsnummer: WO 2002/100364

(56) Entgegenhaltungen:
- DE-A- 2 852 156
- DE-A- 4 102 907
- DE-A- 19 959 318
- US-A- 4 371 370
- US-A- 6 024 769

## Beschreibung

Die Erfindung betrifft Mittel zum Färben keratinischer Fasern, die verbrückte m-Phenylendiaminderivate als Kupplerkomponente in Kombination mit mindestens einer Entwicklerkomponente enthalten, ein Verfahren zum Färben keratinischer Fasern mit diesen Mitteln, die Verwendung der verbrückten m-Phenylendiaminderivate zur Färbung keratinischer Fasern sowie neue verbrückte m-Phenylendiaminderivate.

Keratinfasern, insbesondere das menschliche Haar, werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintritt. Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z.B. Dauerwellenflüssigkeiten. Schließlich sollen sie - falls als Haarfärbemittel zur Anwendung kommend - die Kopfhaut möglichst wenig anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie dennoch nicht den individuellen Wünschen der einzelnen Person entspricht und rückgängig gemacht werden soll.

Für Haarfärbe- und Tönungsmittel kommt insbesondere den Farbnuancen im Rot- und Braunbereich eine erhebliche Bedeutung zu, da sie zur Erzeugung natürlich wirkender Haarfärbungen zwingend erforderlich sind. Oxidationsfärbemittel im Rot- und Braunbereich, beispielsweise zugänglich mit der Kombination 3,5-Diamino-2-methoxytoluol und p-Toluylendiamin (PDT), sind zwar hervorragend bezüglich ihrer Farbintensität, anwendungstechnisch weisen sie jedoch noch einige Mängel auf.

Aus DE 28 52 156 waren bereits Bis-(2,4-diaminophenoxy)-alkane bekannt, die mit vielen bekannten Entwicklerverbindungen, insbesondere mit p-Toluylendiamin, zu intensiven blauen Oxidationsfarbstoffen kuppeln.

Die DE 28 52 156 offenbart Haarfärbemittel, mit denen sich die gewünschten Farbnuancen in ausreichender Intensität und Echtheit herstellen lassen, die ein gutes Aufziehvermögen besitzen, die beständig gegen Wärme, Licht, Reibung und chemischen Reduktionsmitteln sind sowie toxikologisch und dermatologisch unbedenklich sind. Mit unterschiedlichen Entwicklerkomponenten lassen sich unter Einsatz der zweikernigen Kupplerkomponenten Bis-(2,4-diaminophenoxy)-alkane verschiedene Blautöne erzielen.

Die DE 41 02 907 offenbart Haarfärbemittel mit der Kupplerkomponente Bis-(2,5-diaminophenoxy)-alkan, die Blautöne hervorrufen.

Völlig überraschend wurde nun herausgefunden, daß die Umsetzung der mit diesen Substanzen eng verwandten Bis-(2,4-diamino-3,6-dialkylphenoxy)-alkane und Bis-(2,4-diamino-6-alkylphenoxy)-alkane mit vielen üblichen Entwicklersubstanzen, beispielsweise p-Toluylendiamin, zu intensiven roten Farbtönen mit sehr guter Wasch- und Lichtechtheit führt. Insbesondere das 1,3-Bis-(2,4-diamino-6-methylphenoxy)-propan hat sich nicht nur als ausgezeichnete Kupplerkomponente für Oxidationshaarfarben im Rot-Bereich erwiesen, sondern verfügt über herausragende Eigenschaften in toxikologischer Hinsicht.

Ein erster Gegenstand der Erfindung ist daher ein Mittel zum Färben von Keratinfasern, insbesondere von menschlichen Haaren, das in einem zum Färben geeigneten Träger als Oxidationsfarbstoffvorprodukte mindestens eine Entwicklerkomponente und mindestens eine Kupplerkomponente enthält, wobei die Kupplerkomponente ein verbrücktes Phenylendiaminderivat der Formel (I) oder dessen physiologisch verträgliches Salz ist, 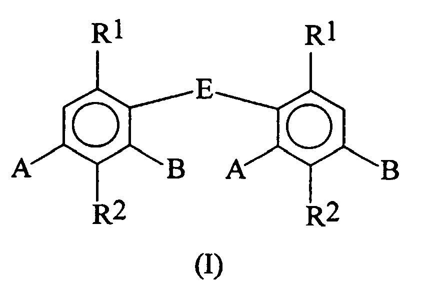 wobei
- A und B unabhängig voneinander stehen für eine -NH₂, -NHR oder eine -NR^{a}R^{b}-Gruppe, worin R, R^{a} und R^{b} unabhängig voneinander einen C₁-C₄-Alkylrest, einen C₂-C₄-Mono- oder Polyhydroxyalkylrest bedeuten können,
- R¹ und R² stehen unabhängig voneinander für Halogen, eine C₁-C₄-Alkylgruppe, eine C₁-C₄-Hydroxyalkylgruppe, eine C₂-C₄-Dihydroxyalkylgruppe , eine C₁-C₄-Alkoxygruppe oder eine C₁-C₄-Hydroxyalkoxygruppe, mit der Maßgabe, daß R² zusätzlich für Wasserstoff stehen kann und
- E steht für

| | |
|---|---|
| -O-(CR³R ⁴)ₘ-O- | mit: R³, R⁴ stehen unabhängig voneinander für Wasserstoff, eine C₁-C₄-Alkylgruppe, eine C₁-C₄-Hydroxyalkylgruppe, eine C₂-C₄-Dihydroxyalkylgruppe, eine C₁-C₄-Alkoxygruppe oder eine C₁-C₄-Hydroxyalkoxygruppe und |
| -O-(CₙH₂ₙ₋ₓ(OH)ₓ)-O- | m steht für eine ganze Zahl von 1 bis 10, mit: n steht für eine ganze Zahl von 3 bis 10 und x steht für eine Zahl von 1 bis 8, mit der Maßgabe, daß n größer als x ist, |
| 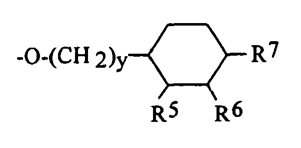 | mit: y steht für eine ganze Zahl von 1 bis 5, einer der drei Reste R⁵, R⁶ und R⁷ steht für die Gruppierung -(CH₂)_{y}-O- und die beiden anderen Reste stehen unabhängig voneinander für Wasserstoff, eine Hydroxygruppe oder eine Aminogruppe, |
| 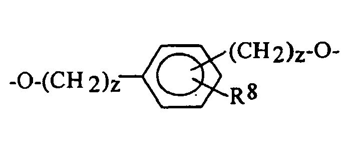 | mit: z steht für eine ganze Zahl von 1 bis 5 und R⁸ steht für Wasserstoff, Halogen, eine C₁-C₄-Alkylgruppe, eine C₁-C₄-Hydroxyalkylgruppe, eine C₂-C₄-Dihydroxyalkylgruppe oder eine C₁-C₄-Hydroxyalkoxygruppe. |

Diese Verbindungen lassen sich mit literaturbekannten, organischen Synthesemethoden herstellen. Bezüglich der detaillierten Herstellungsvorschrift wird ausdrücklich auf die im Beispielteil beschriebenen Synthesebeispiele verwiesen.

Da es sich bei allen erfindungsgemäßen Substanzen um Diamino-Verbindungen handelt, lassen sich aus diesen in üblicher Weise die entsprechenden Säureadditionssalze herstellen. Alle Aussagen dieser Schrift und demgemäß der beanspruchte Schutzbereich beziehen sich daher sowohl auf die in freier Form vorliegenden verbrückten m-Phenylendiaminderivate gemäß Formel (I) als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Erfindungsgemäß bevorzugte Salze gemäß Formel (I) sind die Hydrochloride.

Beispiele für C₁-C₄-Alkylgruppen in den erfindungsgemäßen Verbindungen sind Methyl, Ethyl, n-Propyl, Isopropyl, n-Butyl, Isobutyl und tert-Butyl. Bevorzugte Alkylgruppen sind Methyl und Ethyl, Methyl ist eine besonders bevorzugte Alkylgruppe. Bevorzugte C₂-C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe. Erfindungsgemäße Beispiele für C₂-C₄-Dihydroxyalkylgruppen und C₁-C₄-Hydroxyalkoxygruppen sind Dihydroxybutyl und 2-Hydroxy-3-methoxypropyl. Als Halogensubstituenten eignen sich erfindungsgemäß bevorzugt Chlor, Brom und Iod, besonders bevorzugt sind Chlor und Brom.

Bevorzugt sind Verbindungen der Formel (I), in denen die Gruppen A und B für -NH₂-Gruppen stehen.

Ebenfalls bevorzugt sind Verbindungen der Formel (I), bei denen der Rest R¹ eine Methylgruppe ist.

Weiterhin sind solche Verbindungen der Formel (I) bevorzugt, bei denen R² Wasserstoff oder eine Methylgruppe ist, wobei R² ganz besonders bevorzugt für Wasserstoff steht.

Bevorzugte Verbindungen der Formel (I) sind auch solche, bei denen die Reste R³ und R⁴ Wasserstoff sind.

Die ganzen Zahlen m und n sind für bevorzugte Verbindungen der Formel (I) 3 oder 4 und x ist 1 oder 2.

Unter den Verbindungen der Formel (I), die in der Brücke Cycloalkyl- oder Xylol-Derivate enthalten, sind solche bevorzugt, bei denen y und z ganze Zahlen zwischen 1 und 3 sind.

Bevorzugte Verbindungen der Formel (I) sind auch solche, bei denen einer der Reste R⁵ bis R⁷ für eine -(CH₂)_{y}-O-Gruppierung und die beiden anderen für Wasserstoff stehen.

Bevorzugte Verbindungen der Formel (I) sind auch solche, bei denen der Rest R⁸ für Wasserstoff steht.

Ganz besonders bevorzugte Verbindungen der Formel (I) sind erfindungsgemäß 1,3-Bis-(2,4-diamino-3,6-dimethylphenoxy)-propan und 1,3-Bis-(2,4-diamino-6-methylphenoxy)-propan und deren physiologisch verträgliche Salze.

Als weitere zwingende Komponente enthalten die erfindungsgemäßen Mittel eine Entwicklerkomponente. Dafür werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.
Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 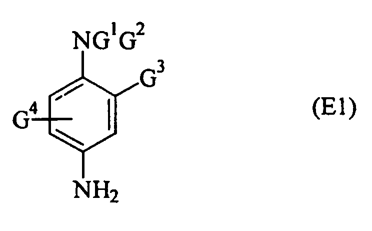 wobei
- G¹ steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkylrest, einen C₁-C₄-Monohydroxyalkykest, einen C₂-C₄-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom, Jod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest,
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁-C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Ein Beispiel für eine C₂-C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Toluylendiamin, p-Phenylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 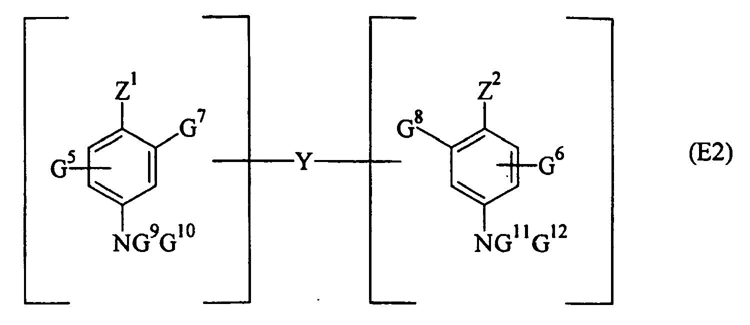 wobei:
- Z¹ und Z² stehen unabhängig voneinander für einen Hydroxyl- oder NH₂-Rest, das gegebenenfalls durch einen C₁-C4-Alkylrest, durch einen C₁-C₄-Hydroxyalkylrest und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄- Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.
Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'-methylphenyl)-ethylendiamin, Bis-(2-hydroxy-5-aminophenyl)-methan, 1,4-Bis-(4'-aminophenyl)-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 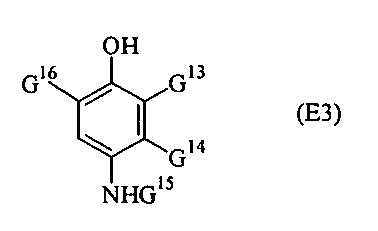 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff oder Halogenatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄-alkylrest, einen C₁-C₄-Aminoalkylrest oder einen C₁-C₄-Cyanoalkylrest,
- G¹⁵ steht für Wasserstoff, einen C₁-C₄-Alkylrest, einen C₁-C₄-Monohydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methylphenol, 4-Amino-3-fluorphenol, 2-Hydroxy-methylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethylaminomethyl)phenol, 4-Amino-2-(α,β-Dihydroxyethyl)-phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-Dihydroxyethyl)-phenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen. Bevorzugt werden erfindungsgemäß Pyrimidin oder Pyrazolderivate.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 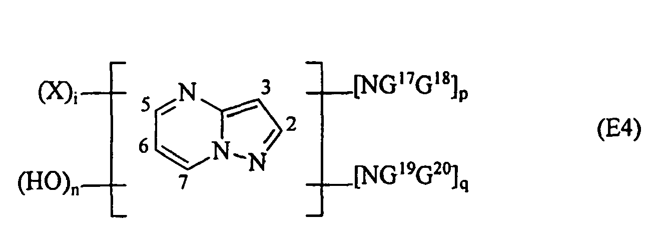 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest, einen C₁-C₄-Aminoalkylrest, das gegebenenfalls durch einen Acetyl-Ureid- oder Sulfonyl-Rest geschützt sein kann, einen (C₁-C₄)-Alkylamino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄-Alkylrest, einen Aryl-Rest, einen C₁-C₄-Hydroxyalkylrest, einen C₂-C₄-Polyhydroxyalkylrest, einen C₁-C₄-Aminoalkylrest, einen (C₁-C₄)-alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine S ul fonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

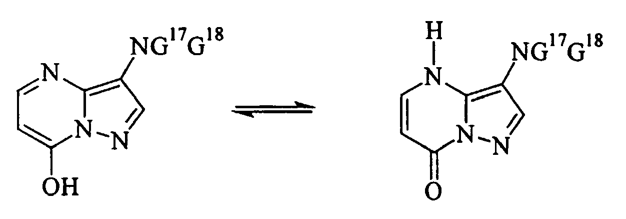

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß bevorzugte Entwicklerkomponenten sind Pyrimidinderivate, Pyrazolderivate, p-Aminophenolderivate und p-Diaminobenzolderivate.

Besonders bevorzugte Entwicklerkomponenten sind erfindungsgemäß p-Phenylendiamin, p-Toluylendiamin, 2-(β-Hydroxyethyl)-p-phenylendiamin, N,N-Bis-(β-hydroxyethyl)-p-phenylendiamin, 2-(α,β-Dihydroxyethyl)-p-phenylendiamin, N,N'-bis-(β-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan, 1,10-Bis-(2',5'-diaminophenyl)-1,4,7,10-tetraoxadecan, p-Aminophenol, 4-Amino-3-methylphenol, 4-Amino-3-fluorphenol, 4-Amino-2-aminomethylphenol, 4-Amino-2-((diethylamino)methyl)phenol, 2-Amino-4-methylphenol, 2-Amino-4-chlorphenol, 4-Amino-2-(α,β-Dihydroxyethyl)-phenol, (2,5-Diaminopyridin, 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 1-(2'-Hydroxy-5'-aminobenzyl)-imidazolidin-2-on und 4,5-Diamino-1-(2'-hydroxyethyl)pyrazol.

Ganz besonders bevorzugte Entwicklerkomponenten ist erfindungsgemäß p-Toluylendiamin.

Weiterhin ganz besonders bevorzugte Entwicklerkomponenten sind p-Phenylendiamin, 2-(β-Hydroxyethyl)-p-phenylendiamin, N,N-Bis-(β-hydroxyethyl)-p-phenylendiamin, N,N'-bis-(β-Hydroxyethyl)-N,N'-bis-(4'-aminophenyl)-1,3-diamino-propan-2-ol und 2,5-Diaminopyridin.

Allein mit einer Entwicklerkomponente oder einer speziellen Kuppler/Entwickler-kombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Als weitere Kupplerkomponenten werden in der Regel m-Phenylendiaminderivate, Naphthole, Resorcin und Resorcinderivate, Pyrazolone, und m-Aminophenole verwendet.

Erfindungsgemäß bevorzugte zusätzliche Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, '1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kupplerkomponenten sind 2-Amino-3-hydroxy-5-chlorpyridin, 2,6-Dihydroxy-3,4-dimethylpyridin, 2,6-Bis-(2-hydroxyethylamino)-toluol, 3-Amino-2,4-dichlorphenol, 3-Amino-2-chlor-6-methylphenol, 5-Amino-4-chlor-2-methylphenol, 5-(2`-Hydroxyethyl)amino-2-methylphenol, 5-Amino-2-methylphenol, 2-Amino-3-hydroxypyridin, 3-Amino-2-methylamino-6-methoxy-pyridin, 2-Methylresorcin, 4-Chlorresorcin 2,4-Diaminophenoxyethanol, 1,3-Bis-(2,4-diaminophenoxy)propan, Resorcin, m-Aminophenol, 3,5-Diamino-2,6-dimethoxypyridin, 1,7-, 2,7- und 1,5-Dihydroxynaphthalin sowie 4-Hydroxyindol und 6-Hydroxyindol.

Ganz besonders bevorzugte weitere Kupplerkomponenten sind 5-Amino-2-methylphenol, 2-Methylresorcin, 4-Chlorresorcin 2,4-Diaminophenoxyethanol, 1,3-Bis-(2,4-diaminophenoxy)propan und 2,4-Dichlor-3-aminophenol.

Die erfindungsgemäßen Haarfärbemittel enthalten sowohl die Entwicklerkomponenten als auch die Kupplerkomponenten bevorzugt in einer Menge von 0,005 bis 10 Gew.-% vorzugsweise von 0,1 bis 5 Gew.-% jeweils bezogen auf das gesamte Oxidationsfärbemittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem MolVerhältnis von 1:0,5 bis 1:3, insbesondere 1:1 1 bis 1:2, enthalten sein können.

Weiterhin können die erfindungsgemäßen Mittel Vorstufen naturanaloger Farbstoffe enthalten. Dafür werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 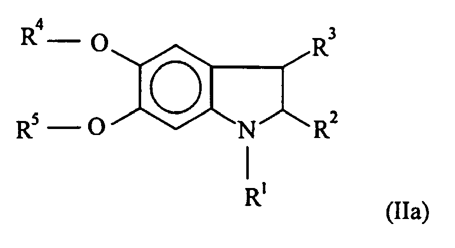 in der unabhängig voneinander
R¹ steht für Wasserstoff, eine C₁-C₄-Alkylgruppe, eine C₁-C₄-Hydroxyalkylgruppe, eine C₃-C₆-Cycloalkylgruppe oder eine C₂-C₄-Alkenylgruppe, insbesondere eine Vinyl- oder Allylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 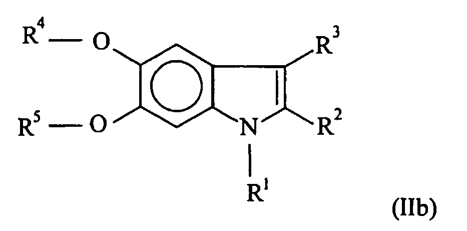 in der unabhängig voneinander
R¹ steht für Wasserstoff, eine C₁-C₄-Alkylgruppe, eine C₁-C₄-Hydroxyalkylgruppe, eine C₃-C₆-Cycloalkylgruppe oder eine C₂-C₄-Alkenylgruppe, insbesondere eine Vinyl- oder Allylgruppe
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.
Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hybrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin; 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

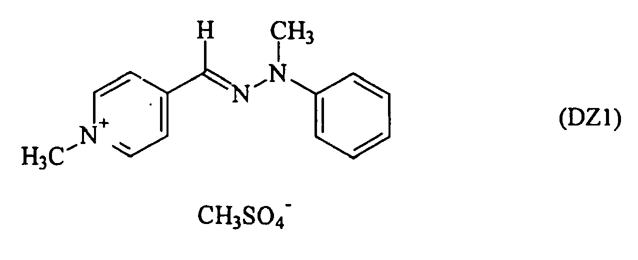

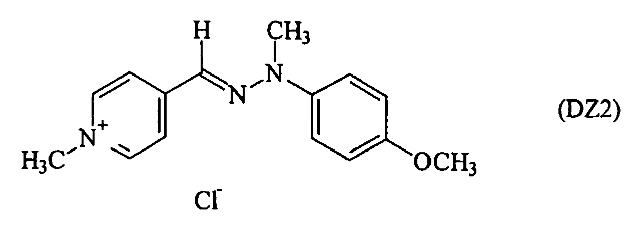

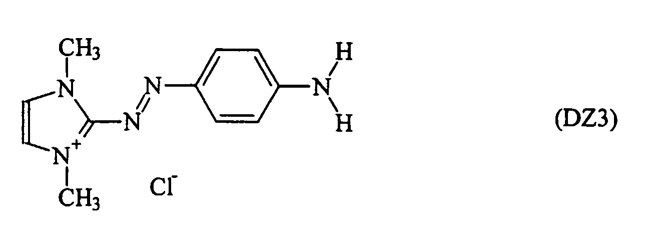

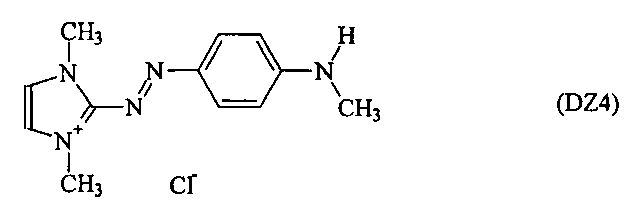

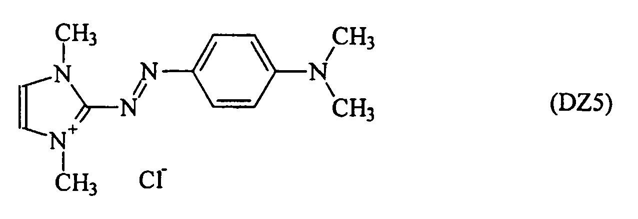

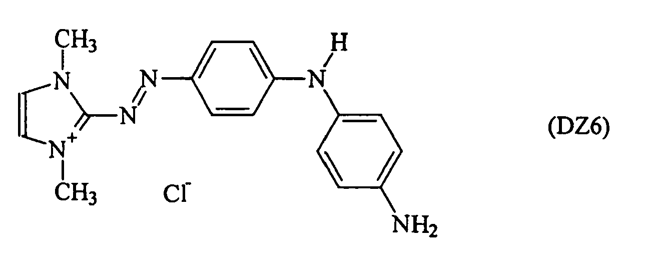

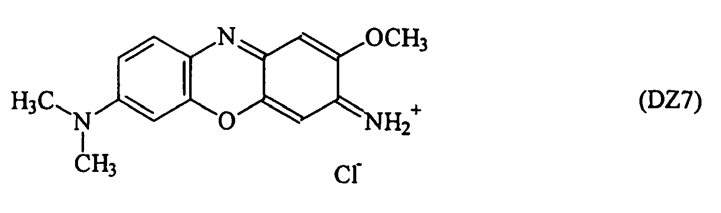

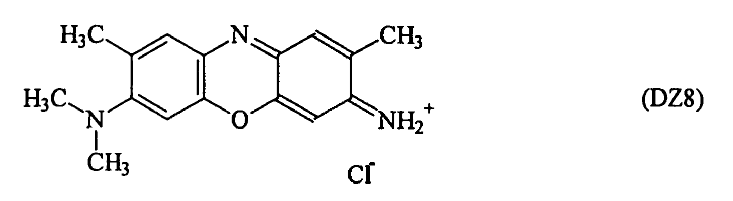

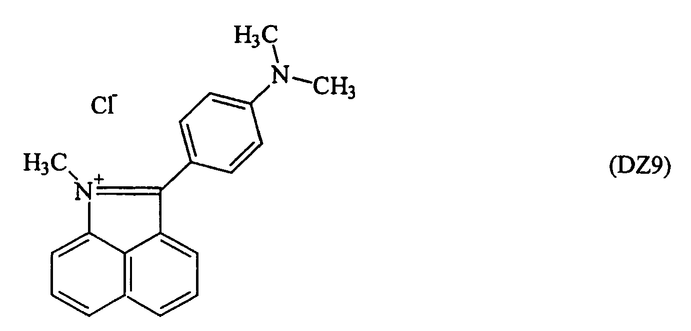

Die Verbindungen der Formeln (DZ1), (DZ3) und (DZ5) sind ganz besonders bevorzugte kationische direktziehende Farbstoffe der Gruppe (c).

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe enthalten, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind.

Die erfindungsgemäßen Mittel enthalten Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.
Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R'O-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R'
- im wesentlichen aus C₈- und C₁₀-Alkylgruppen,
- im wesentlichen aus C₁₂- und C₁₄-Alkylgruppen,
- im wesentlichen aus C₈-C₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammonium-glycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethyl-ammoniumchlorid, Lauryldimethylammoniumchlorid, Lauryldimethylbenzylammonium-chlorid und Tricetylmethylammoniumchlorid, sowie die unter den INCI-Bezeichnungen Quatemium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®} 400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®} 100 (Poly(dimethyldiallylammoniumchlorid)), Merquat^{®}550 (Dimethyldiallylammoniumchlorid-Acrylamid-Copolymer) und Merquat^{®} 280 (Dimethyldiallyl-ammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.
Besonders bevorzugt sind kationische Polymere der vier erstgenannten Gruppen, ganz besonders bevorzugt sind Polyquaternium-2, Polyquatemium-10 und Polyquatemium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist ein Verfahren zur Färbung von Keratinfasern, insbesondere Haaren, unter Einsatz eines erfindungsgemäßen Oxidationsfärbemittels. Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase, Ascorbat-Oxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Ein dritter Gegenstand der Erfindung ist ebenso die Verwendung von Verbindungen gemäß Formel (I) als Kupplerkomponente in Oxidationsfärbemitteln zur Färbung von keratinischen Fasern, insbesondere von Haaren.

Neu und somit ein vierter Gegenstand der Erfindung sind die Verbindungen entsprechend Formel (I) gemäß Anspruch 1, die durch folgende Substituentenkombinationen gekennzeichnet sind:

| | **A=B** | **E** | **R¹** | **R²** |
|---|---|---|---|---|
| **1** | -NH₂ | -O-(CH₂)₃-O- | -CH₃ | -CH₃ |
| **2** | -NH₂ | -O-(CH₂)₃-O- | -CH₃ | -H |

Die folgenden Beispiele sollen den Gegenstand der vorliegenden Anmeldung verdeutlichen.

### Beispiele

### 1. Herstellungsbeispiele

### 1.1 1,3-Bis-(2,4-diamino-3,6-dimethylphenoxy)-propan*4HCl

### a) 1,3-Bis-(3,6-dimethylphenoxy)-propan

Zu einer Mischung bestehend aus 74 g (0.6 mol) 2,5-Dimethylphenol , 180 ml Ethanol und 36.6 g (0.6 mol) KOH wurden 61.2 g (0.3 mol) 1,3-Dibrompropan getropft. Es wurde drei Stunden unter Rückfluß gekocht und die Reaktionsmischung anschließend in 2 1 Eiswasser gegossen. Das Produkt wurde mit Methyl-t-butylester extrahiert und konnte in Form eines hellgelben Öls erhalten werden.
Ausbeute: 69 g (40% d. Th.)
Das Molgewicht der Substanz wurde massenspektrometrisch bestimmt, der Molpeak lag bei 284. (Theorie: 284).

### b) 1,3-Bis-(2,4-dinitro-3,6-dimethylphenoxy)-propan

10 ml einer 10%igen Salpetersäurelösung wurden bei -10 - 0°C vorgelegt und mit 5.7 g (0.02 mol) 1,3-Bis-(3,6-dimethylphenoxy)-propan versetzt. Nach einstündigem Rühren unter Eiswasserkühlung wurde die Reaktionsmischung auf Eiswasser gegossen und der entstandene Niederschlag abgesaugt. Bei dem Produkt handelte es sich um gelbe Kristalle.
Ausbeute: 9.3 g (100% d. Th.)
Das Molgewicht der Substanz wurde massenspektrometrisch bestimmt, der Molpeak lag bei 464. (Theorie: 464).

### c) 1,3-Bis-(2,4-diamino-3,6-dimethylphenoxy)-propan*4HCl

9.3 g 1,3-Bis-(2,4-dinitro-3,6-dimethylphenoxy)-propan, gelöst in 400 ml Ethanol, wurden mit 0.6 g eines Palladium-auf-Kohle-Katalysators (5% Pd) bei Raumtemperatur hydriert. Nach beendeter Wasserstoff-Aufuahme wurde die Lösung mit 100 ml verdünnter HCl versetzt und vom Katalysator abfiltriert. Anschließend wurde die Lösung bis zur Trockene eingeengt und das Produkt in Form hellbeiger Kristalle isoliert.
Ausbeute: 9 g (91.8% d. Th.)
Smp.: > 180°C (dann Zersetzung)
Das Molgewicht der Substanz wurde massenspektrometrisch bestimmt, der Molpeak lag bei 490. (Theorie: 490).

### 1.2 1,3-Bis-(2,4-diamino-6-methylphenoxy) propan*4HCl

### a) 1,3-Bis-(2,4-dinitro-6-methylphenoxy)-propan

Eine Mischung bestehend aus 20 g 4,6-Dinitro-o-kresol und 50 ml Methylglycol wurde auf 60°C erwärmt. 6.4 g Kaliumcarbonat wurden portionsweise hinzugefügt und die Mischung zum Sieden erhitzt. Anschließend wurden 9.3 g 1,3-Dibrompropan zugetropft, noch drei Stunden gerührt und auf Raumtemperatur abgekühlt. Das Produkt wurde abgesaugt und lag in Form hellbrauner Kristalle vor.
Smp.: 113°C

### b) 1,3-Bis-(2,4-diamino-6-methylphenoxy)-propan*4HCl

5 g 1,3-Bis-(2,4-dinitro-6-methylphenoxy)-propan in 100 ml Ethanol wurden mit 0.4 g eines Katalysators (5% Pd auf Kohle) bei Raumtemperatur und einem Druck von 1 atü hydriert. Nach beendeter Wasserstoff-Aufnahme wurde der Katalysator abfiltriert und die Lösung mit 10 %iger HCl angesäuert. Das nach dem Einengen entstandene Produkt wurde in Form farbloser Kristalle isoliert.
Smp.: >250°C

### 2. Anwendungsbeispiele

### 1. Ausfärbungsbeispiel

Es wurde eine Basis-Creme der folgenden Zusammensetzung hergestellt:

| | |
|---|---|
| Talgfettalkohol (C₁₆-C₁₈) | 17.0 g |
| Kokosfettalkohol (C₁₂-C₁₈) | 4.0 g |
| Natriumlaurylethersulfat (28%ige Lösung) | 40.0 g |
| Cocoamidopropyl Betaine (30%ige Lösung) | 25.0 g |
| Cetyl-/Stearylalkohol-poly(20EO)glycolether | 1.5 g |
| Wasser | 12.5 g |

Es wurden Färbecremes der folgenden Zusammensetzungen hergestellt:

| | |
|---|---|
| Basiscreme | 50.0 g |
| Entwicklerkomponente | 2.5 mmol |
| Kupplerkomponente | 2.5 mmol |
| Na₂SO₃ (Inhibitor) | 1.0 g |
| (NH₄)₂SO₄ | 1.0 g |
| Konz. NH₃-Lösung | ad pH=10 |
| Wasser | ad 100 g |

Für die Ausfärbungen wurden folgende Oxidationsfarbstoffvorprodukte eingesetzt:
Entwicklerkomponenten:
   E1: p-Toluylendiamin
   E2: 2,5-Diaminophenylethanol
   E3: 1,3-N,N'-Bis-(2'-hydroxyethyl)-N,N'-bis-(4-aminophenyl)-diaminopropan-2-ol
   E4: N,N'-Bis-(2'-hydroxyethyl)amino-p-phenylendiamin
   E5: p-Phenylendiamin
   E6: 2,5-Diaminopyridin
Kupplerkomponenten:
   K1: 1,3-Bis-(2,4-diamino-3,6-dimethylphenoxy)-propan (erfindungsgemäß)
   K2: 1,3-Bis-(2,4-diaminophenoxy)-propan
   K3: 2,4-Dichlor-3-aminophenol
   K4: 2,4-Diaminophenoxyethanol
   K5: 1,3-Bis-(2,4-diamino-6-methylphenoxy)-propan (erfindungsgemäß)

Die Bestandteile wurden der Reihe nach miteinander vermischt. Nach Zugabe der Oxidationsfarbstoffvorprodukte, des Inhibitors und des Ammoniumsulfats wurde mit konzentrierter Ammoniaklösung der pH-Wert der Emulsion auf 10 eingestellt, dann wurde mit Wasser auf 100 g aufgefüllt.
Wenn zwei Entwickler oder zwei Kuppler eingesetzt wurden, wurden von jeder Verbindung 1,25 mmol verwendet, so daß die Gesamtmenge an Entwicklerkomponenten bzw. Kupplerkomponenten 2,5 mmol pro 100 g Färbecreme betrug.

Die Entwicklung der Färbung wurde mit 1 Gew.%-iger Wasserstoffperoxidlösung durchgeführt. Hierzu wurden 100 g der Färbecreme mit 50 g H₂O₂-Lösung (1%ig) gemischt.

Die gebrauchsfertigen Färbeansätze wurden dann auf 5 cm lange Strähnen standardisierten, zu 80 % ergrauten Menschenhaares (Fa. Kerling) aufgetragen. Nach 30 Minuten Einwirkungszeit bei 32°C wurde das Haar mit Wasser gespült, mit einem üblichen Shampoo ausgewaschen, erneut gespült und getrocknet.

Die Ergebnisse der Färbeversuche sind der folgenden Tabelle zu entnehmen:

| | Kupplerkomponente | Entwicklerkomponente | Farbton des gefärbten Haares |
|---|---|---|---|
| 1 | K1 | E1 | Englischrot |
| 2 | K1 | E2 | Terracotta |
| 3 | K1 | E3 | Rotbraun |
| 4 | K1 | E4 | Pompejanischrot |
| 5 | K1 + K2 | E1 | Schwarzblau |
| 6 | K1 + K3 | E1 | Rotbraun |
| 7 | K1 + K4 | E2 | Marineblau |
| 8 | K5 | E5 | Rotbraun |
| 9 | K5 | E1 | Persischrot |
| 10 | K5 | E6 | Rotbraun |

### II. Ausfärbungsbeispiel

Es wurden Färbecremes mit den Farbstoffvorprodukten F0 - F41 wie folgt hergestellt.

| | Rezeptur-Nummer / Mengenangaben in Gew.% | | | | | | | |
|---|---|---|---|---|---|---|---|---|
| | **1** | **2** | **3** | **4** | **5** | **6** | **7** | **8** |
| Fettalkoholgemisch C₁₂-C₁₈ | 6.0 | 6.0 | 6.0 | 6.0 | 6.0 | 6.0 | 6.0 | 6.0 |
| Eumulgin B2¹ | 0.2 | 0.2 | 0.2 | 0.2 | 0.2 | 0.2 | 0.2 | 0.2 |
| Eumulgin B1² | 0.2 | 0.2 | 0.2 | 0.2 | 0.2 | 0.2 | 0.2 | 0.2 |
| Texapon NSO³ | 10.0 | 10.0 | 10.0 | 10.0 | 10.0 | 10.0 | 10.0 | 10.0 |
| Dehyton K⁴ | 6.3 | 6.3 | 6.3 | 6.3 | 6.3 | 6.3 | 6.3 | 6.3 |
| Polymer JR 400⁵ | 1.0 | 1.0 | 1.0 | 1.0 | 1.0 | 1.0 | 1.0 | 1.0 |
| Lamesoft P0 65⁶ | 2.0 | 2.0 | 2.0 | 2.0 | 2.0 | 2.0 | 2.0 | 2.0 |
| Eutanol G⁷ | 1.0 | 1.0 | 1.0 | 1.0 | 1.0 | 1.0 | 1.0 | 1.0 |
| Akyposoft 45 NV⁸ | 2.0 | 2.0 | 2.0 | 2.0 | 2.0 | 2.0 | 2.0 | 2.0 |
| Na₂SO₃ | 0.3 | 0.3 | 0.3 | 0.3 | 0.3 | 0.3 | 0.3 | 0.3 |
| (NH₄)₂SO₄ | 0.5 | 0.5 | 0.5 | 0.5 | 0.5 | 0.5 | 0.5 | 0.5 |
| F0 (erfindungsgemäß) | 1.05 | 0.74 | 0.37 | 0.09 | 0.05 | 0.1 | 0.05 | 0.4 |
| F1 | 0.6 | - | - | - | 0.09 | - | 0.93 | 1.0 |
| F2 | - | - | 0.42 | - | 0.04 | - | - | 0.3 |
| F3 | - | - | 0.40 | - | - | - | - | 0.3 |
| F4 | - | 0.68 | - | - | 0.04 | 0.2 | - | - |
| F5 | 0.09 | - | - | - | - | - | - | - |
| F6 | 0.3 | - | - | - | 0.01 | - | 0.01 | - |
| F6 | - | - | 0.5 | - | - | - | 0.01 | - |
| F8 | - | - | 0.56 | - | 0.03 | 2.30 | 0.01 | - |
| F9 | - | - | 0.09 | - | - | 0.05 | - | - |
| F10 | - | - | - | - | 0.8 | - | - | - |
| F11 | - | - | - | - | 0.05 | - | - | - |
| F12 | - | - | - | 1.05 | 0.3 | - | - | - |
| F13 | - | - | 0.03 | - | - | - | - | - |
| F14 | 0.4 | 0.12 | - | - | 0.04 | - | 0.047 | 0.5 |
| F15 | - | - | 0.07 | - | 0.22 | - | 0.023 | 0.08 |
| F16 | - | - | 0.08 | - | - | - | 0.063 | - |
| F17 | - | - | - | 0.03 | - | - | 0.02 | - |
| F18 | - | - | - | 0.02 | - | - | 0.01 | - |
| F19 | - | - | - | - | 0.02 | - | 0.05 | - |
| F20 | - | - | 0.09 | - | 0.3 | - | 0.08 | - |
| F21 | - | - | - | - | 0.01 | - | 0.015 | 0.14 |
| F22 | - | - | 0.2 | - | 0.03 | - | 0.22 | - |
| F23 | - | - | 0.38 | - | - | - | - | - |
| F24 | - | - | - | - | - | - | 0.03 | - |
| F25 | - | - | - | - | 0.2 | - | 0.07 | - |
| F26 | - | - | - | 0.8 | - | - | - | 0.01 |
| F27 | - | - | - | - | - | - | - | 0.01 |
| F28 | - | - | - | 0.12 | - | - | - | - |
| F29 | - | - | - | 0.04 | - | - | - | 0.01 |
| F30 | - | - | - | 0.04 | - | - | - | - |
| F31 | - | - | - | - | 0.03 | - | - | 0.05 |
| F32 | - | - | 0.08 | - | - | 0.1 | - | - |
| F33 | - | - | - | - | 0.02 | - | - | - |
| F34 | - | - | - | - | - | 1.7 | 0.005 | 0.03 |
| F35 | - | - | - | - | - | 0.1 | - | 0.04 |
| F36 | - | - | - | - | - | 0.05 | - | - |
| F37 | 0.02 | - | - | - | - | 0.1 | - | - |
| F38 | - | - | - | - | - | 0.05 | - | - |
| F39 | - | - | - | - | - | 0.05 | - | - |
| F40 | - | - | - | - | - | 0.2 | - | - |
| F41 | - | - | - | - | 0.15 | - | - | - |
| Ammoniaklösung (25 %ig) | ad pH 10 | | | | | | | |
| Wasser | ad 100 | | | | | | | |

| | | | | | | | | |
|---|---|---|---|---|---|---|---|---|
| ¹ Cetylstearylalkohol+12-Ethylenoxid, (INCI-Bezeichnung: CETEARETH-12) (COGNIS) ² Cetylstearylalkohol+20-Ethylenoxid, (INCI-Bezeichnung: CETEARETH-20) (COGNIS) ³ Laurylethersulfat-Natrium-Salz, (INCI-Bezeichnung: SODIUM LAURETH SULFATE) (COGNIS) ⁴ N,N-Dimethyl-N(C₈₋₁₈-kokosamidopropyl)ammoniumacetobetain, (INCI-Bezeichnung: AQUA (WATER), COCAMIDOPROPYL BETAINE) (COGNIS) ⁵ Hydroxyethylcellulose-quaterniert, (INCI-Bezeichnung: POLYQUATERNIUM-10) (AMERCHOL) ⁶ Alkylpolyglucosid-Ölsäuremonoglycerid-Gemisch, (INCI-Bezeichnung: COCO-GLUCOSIDE, GLYCERYL OLEATE, AQUA (WATER) (COGNIS) ⁷ 2-Octyldodecanol (COGNIS) ⁸ C₁₂₋₁₄-Fettalkohol-4.5-EO-Essigsäure-Natrium-Salz, (INCI-Bezeichnung: SODIUM LAURETH-6 CARBOXYLATE) (KAO) | | | | | | | | |

Das Farbstoffvorprodukt **F0** entspricht dem erfindungsgemäßen Farbstoffvorprodukt 1,3-Bis-(2,4-diamino-6-methylphenoxy)-propan * 4 HCl.

Schlüssel des Verbindungscodes **F1** bis **F41**:

| | | | |
|---|---|---|---|
| **F1** | p-Toluylendiaminsulfat | **F2** | N,N-Bis-(2'-hydroxyethyl)-p-phenylendiaminsulfat |
| **F3** | p-Phenylendiamin*2 HCl | **F4** | 2-(2'-Hydroxyethyl)-p-phenylendiaminsulfat |
| **F5** | p-Aminophenol | **F6** | 4-Amino-3-methylphenol |
| **F7** | 4-Amino-2-[(diethylamino)methyl]-phenol*2 HCl | **F8** | Bis-(5-amino-2-hydroxyphenyl)-methan *2 HCl |
| **F9** | 2,6-Dichlor-4-aminophenol | **F10** | 2,4,5,6-Tetraaminopyrimidinsulfat |
| **F11** | 4-Hydroxy-2,5,6-triaminopyrimidin sulfat-hydrat | **F12** | 4,5-Diamino-1-(2-hydroxyethyl)-pyrazol-sulfat |
| **F13** | o-Aminophenol | **F14** | 2-Methylresorcin |
| **F15** | Resorcin | **F16** | 4-Chlorresorcin |
| **F17** | 1-Naphthol | | |
| **F19** | 1,5-Dihydroxynaphthalin | **F18** | 2-Methyl-1-naphthol |
| **F21** | m-Aminophenol | **F20** | 2,7-Dihydroxynaphthalin |
| **F23** | 5-Amino-4-chlor-2-methyl-phenol * HCl | **F22** | 2-Amino-5-methylphenol |
| **F25** | 3-Amino-2-chlor-6-methyl-phenol | **F24** | 2-(2'-Hydroxyethyl)amino-5-methylphenol |
| **F27** | 1,3-Bis-(2,4-diaminophenoxy)-propan *4 HCl | **F26** | 2,4-Diaminophenoxyethanol *2 HCl |
| **F29** | 2-Amino-4-(2-hydroxyethylamino)-anisolsulfat | **F28** | 2,6-Bis-(2-hydroxyethylamino)-toluol |
| **F31** | 1-Methyl-2-methoxy-3,5-diaminobenzol *2 HCl | **F30** | 3-Amino-2,4-dichlor-phenol * HCl |
| **F33** | 2,6-Dihydroxy-3,4-dimethyl-pyridin | **F32** | 2-Amino-3-hydroxy-pyridin |
| **F35** | 6-Hydroxyindol | **F34** | 3-Amino-2-methylamino-6-methoxypyridin |
| **F37** | 5,6-Dihydroxyindolin * HBr | **F36** | 3,4-Methylendioxyphenol |
| **F39** | 2-Amino-6-chlor-4-nitrophenol | **F38** | 1-(2-Hydroxyethyl)amino-3,4-methylendioxybenzol |
| **F41** | 4-Amino-2-nitro-diphenylamin-2'-carbonsäure | **F40** | HC Red BN |

Nach Herstellung der Färbecremes wurden diese jeweils mit einer 6%igen Wasserstoffperoxidzubereitung (Marktprodukt Poly Color Brillance) im Verhältnis 1:1 vermischt. 4.0 g der Anwendungszubereitung wurde auf 1.0 g Humanhaar (Kerling, naturweiß) aufgetragen. Nach 30min Einwirkzeit bei 32°C wurde die Strähne gespült, getrocknet und das erhaltene Färberesultat visuell bestimmt. Die erhaltenen Nuancen sind der nachstehenden Tabelle zu entnehmen.

| Rezeptur-Nummer | Nuance |
|---|---|
| 1 | Kupferrot |
| 2 | intensives Rot |
| 3 | intensives Violettbraun |
| 4 | dunkles Violett |
| 5 | Englischrot |
| 6 | Achatbraun |
| 7 | dunkles Aubergine |
| 8 | warmes Dunkelbraun |

## Patentansprüche

1. Mittel zur oxidativen Färbung von Keratinfasern, insbesondere von menschlichen Haaren, enthaltend in einem zum Färben geeigneten Medium als Farbstoffvorprodukte mindestens eine Kupplerkomponente und mindestens eine Entwicklerkomponente, **dadurch gekennzeichnet, daß** die Kupplerkomponente ausgewählt ist aus Verbindungen der allgemeinen Formel (I), 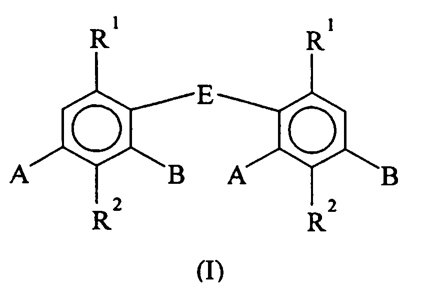 in der
- A und B unabhängig voneinander stehen für eine -NH₂, -NHR oder eine -NR^{a}R^{b}-Gruppe, wobei R, R^{a} und R^{b} unabhängig voneinander einen C₁-C₄-Alkylrest, einen C₂-C₄-Mono- oder Polyhydroxyalkylrest bedeuten können,
- R¹ und R² stehen unabhängig voneinander für Halogen, eine C₁-C₄-Alkylgruppe, eine C₁-C₄-Hydroxyalkylgruppe, eine C₂-C₄-Dihydroxyalkylgruppe , eine C₁-C₄-Alkoxygruppe oder eine C₁-C₄-Hydroxyalkoxygruppe, mit der Maßgabe, daß R² zusätzlich für Wasserstoff stehen kann,
- E steht für
| | |
|---|---|
| -O-(CR³R⁴)ₘ-O- | mit: R³, R⁴ stehen unabhängig voneinander für Wasserstoff, eine C₁-C₄-Alkylgruppe, eine C₁-C₄-Hydroxyalkylgruppe, eine C₂-C₄-Dihydroxyalkylgruppe , eine C₁-C₄-Alkoxygruppe oder eine C₁-C₄-Hydroxyalkoxygruppe und m steht für eine ganze Zahl von 1 bis 10, |
| -O-(CₙH₂ₙ₋ₓ(OH)ₓ)-O- | mit: n steht für eine ganze Zahl von 3 bis 10 und x steht für eine Zahl von 1 bis 8, mit der Maßgabe, daß n größer als x ist, |
| 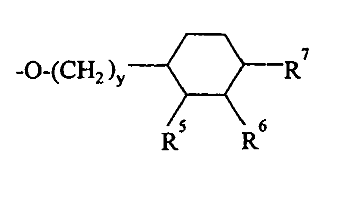 | mit: y steht für eine ganze Zahl von 1 bis 5, einer der drei Reste R⁵, R⁶ und R⁷ steht für die Gruppierung -(CH₂)_{y}-O- und die beiden anderen Reste stehen unabhängig voneinander für Wasserstoff, eine Hydroxygruppe oder eine Aminogruppe, |
| 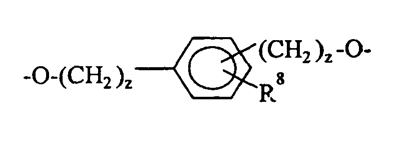 | mit: z steht für eine ganze Zahl von 1 bis 5 und R⁸ steht für Wasserstoff, Halogen, eine C₁-C₄-Alkylgruppe, eine C₁-C₄-Hydroxyalkylgruppe, eine C₂-C₄-Dihydroxyalkylgruppe oder eine C₁-C₄-Hydroxyalkoxygruppe, |
oder deren physiologisch verträglichen Salzen.

2. Mittel gemäß Anspruch 1, **dadurch gekennzeichnet, daß** die Reste A und B -NH₂-Gruppen sind.

3. Mittel gemäß den Ansprüchen 1 oder 2, **dadurch gekennzeichnet, daß** R¹ für eine Methylgruppe steht.

4. Mittel gemäß einem der Ansprüche 1 bis 3, **dadurch gekennzeichnet, daß** R² für Wasserstoff oder eine Methylgruppe steht.

5. Mittel gemäß einem der Ansprüche 1 bis 4, **dadurch gekennzeichnet, daß** die Reste R³ und R⁴ für Wasserstoff stehen.

6. Mittel gemäß einem der Ansprüche 1 bis 5, **dadurch gekennzeichnet, daß** m oder n stehen für 3 oder 4 und x steht für 1 oder 2.

7. Mittel gemäß einem der Ansprüche 1 bis 4, **dadurch gekennzeichnet, daß** y oder z für ganze Zahlen zwischen 1 und 3 stehen.

8. Mittel gemäß einem der Ansprüche 1 bis 4, **dadurch gekennzeichnet, daß** einer der Reste R⁵ bis R⁷ für die Gruppierung -(CH₂)_{y}-O- und die beiden anderen für Wasserstoff stehen.

9. Mittel gemäß einem der Ansprüche 1 bis 4, **dadurch gekennzeichnet, daß** R⁸ für Wasserstoff steht.

10. Mittel gemäß einem der Ansprüche 1 bis 4, **dadurch gekennzeichnet, daß** die Verbindung der allgemeinen Formel (I) 1,3-Bis-(2,4-diamino-3,6-dimethylphenoxy)-propan oder eines seiner physiologisch verträglichen Salze ist.

11. Mittel nach einem der Ansprüche 1 bis 4, **dadurch gekennzeichnet, daß** die Verbindung der allgemeinen Formel (I) 1,3-Bis-(2,4-diamino-6-methylphenoxy)-propan oder eines seiner physiologisch verträglichen Salze ist.

12. Mittel nach einem der Ansprüche 1 bis 11, **dadurch gekennzeichnet, daß** mindestens eine Entwicklerkomponente ausgewählt ist aus p-Phenylendiamin, p-Toluylendiamin, 2-(2'-Hydroxyethyl)-p-phenylendiamin, 2-(α,β-Dihydroxyethyl)-p-phenylendiamin, N,N-Bis-(2'-hydroxyethyl)-p-phenylendiamin, N,N-bis- 2'-hydroxyethyl)amino-p-phenylendiamin, (N,N'-bis-(2'-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan, 1,10-Bis-(2',5'-diaminophenyl)-1,4,7,10-tetraoxadecan, p-Aminophenol, 4-Amino-3-methylphenol, 4-Amino-3-fluorphenol, 4-Amino-2-aminomethylphenol 4-Amino-2-((diethylamino)methyl)phenol, 2-Amino-4-methylphenol, 2-Amino-4-chlorphenol, 4-Amino-2-(α,β-dihydroxyethyl)-phenol, 2,5-Diaminopyridin, 2,4,5,6-Tetraamino-pyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 1-(2'-Hydroxy-5'-aminobenzyl)-imidazolidin-2-on und 4,5-Diamino-1-(2'-hydroxyethyl)pyrazol.

13. Mittel nach einem der Ansprüche 1 bis 12, **dadurch gekennzeichnet, daß** es mindestens eine weitere Kupplerkomponente enthält, die ausgewählt ist aus 2-Amino-3-hydroxy-5-chlorpyridin, 2,6-Dihydroxy-3,4-dimethylpyridin, 2,6-Bis-(2-hydroxyethylamino)-toluol, 3-Amino-2,4-dichlorphenol, 3-Amino-2-chlor-6-methylphenol, 5-Amino-4-chlor-2-methylphenol, 5-(2`-Hydroxyethyl)amino-2-methylphenol, 5-Amino-2-methylphenol, 2-Amino-3-hydroxypyridin, 3-Amino-2-methylamino-6-methoxy-pyridin, 2-Methylresorcin, 4-Chlorresorcin, 2,4-Diaminophenoxyethanol, 1,3-Bis-(2,4-diaminophenoxy)propan, Resorcin, m-Aminophenol, 3,5-Diamino-2,6-dimethoxypyridin, 1,7-, 2,7- und 1,5-Dihydroxynaphthalin sowie 4-Hydroxyindol und 6-Hydroxyindol.

14. Mittel nach einem der Ansprüche 1 bis 13, **dadurch gekennzeichnet, daß** es zusätzlich einen direktziehenden Farbstoff enthält.

15. Mittel nach einem der Ansprüche 1 bis 14, **dadurch gekennzeichnet, daß** es weiterhin ein anionisches, nichtionogenes oder kationisches Polymeres enthält.

16. Mittel nach einem der Ansprüche 1 bis 15, **dadurch gekennzeichnet, daß** es weiterhin mindestens ein Tensid enthält.

17. Mittel nach einem der Ansprüche 1 bis 16, **dadurch gekennzeichnet, daß** sie weiterhin eine emulgierbare Fettkomponente, bevorzugt ein C₁₂-C₂₂-Fettalkoholgemisch enthält.

18. Verfahren zur oxidativen Färbung von Keratinfasern, insbesondere von menschlichen Haaren, **dadurch gekennzeichnet, daß** ein Mittel gemäß einem der Ansprüche 1 bis 15 auf die Fasern bei einer Temperatur zwischen 15 und 40°C aufgetragen und nach einer Einwirkungszeit von 5 bis 45 Minuten wieder abgespült wird.

19. Verwendung von Verbindungen der allgemeinen Formel (I) gemäß einem der Ansprüche 1 bis 15 zur oxidativen Färbung von Keratinfasern.

20. Verbindungen entsprechend Formel (I) gemäß Anspruch 1, die durch folgende Substituentenkombinationen **gekennzeichnet** sind:
| | **A=B** | **E** | **R¹** | **R²** |
|---|---|---|---|---|
| **1** | -NH₂ | -O-(CH₂)₃-O- | -CH₃ | -CH₃ |
| **2** | -NH₂ | -O-(CH₂)₃-O- | -CH₃ | -H |

## Claims

1. A preparation for coloring keratin fibers, more particularly human hair, which contains at least one primary intermediate and at least one secondary intermediate as oxidation dye precursors in a carrier suitable for coloring, **characterized in that** the secondary intermediate is selected from compounds corresponding to formula (1): 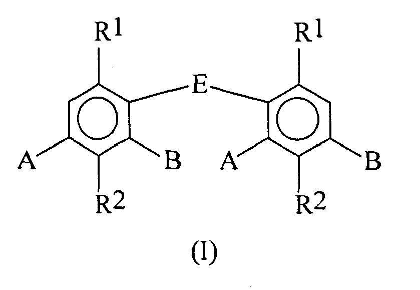 in which
A and B independently of one another represent an -NH₂, -NHR or -NR^{a}R^{b} group where R, R^{a} and R^{b} independently of one another may represent a C₁₋₄ alkyl group, a C₂₋₄ mono- or polyhydroxyalkyl group,
R¹ and R² independently of one another represent halogen, a C₁₋₄ alkyl group, a C₁₋₄ hydroxyalkyl group, a C₂₋₄ dihydroxyalkyl group, a C₁₋₄ alkoxy group or a C₁₋₄ hydroxyalkoxy group, with the proviso that R² may additionally represent hydrogen and
E represents
| | |
|---|---|
| -O-(CR³R⁴)ₘ-O- | where R³ and R⁴ independently of one another represent hydrogen, a C₁₋₄ alkyl group, a C₁₋₄ hydroxyalkyl group, a C₂₋₄ dihydroxyalkyl group, a C₁₋₄ alkoxy group or a C₁₋₄ hydroxyalkoxy group and m is an integer of 1 to 10, |
| -O-(CₙH₂ₙ₋ₓ(OH)ₓ)-O- | where n is an integer of 3 to 10 and x is a number of 1 to 8, with the proviso that n is greater than x, |
| 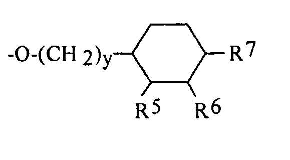 | where y is an integer of 1 to 5, one of the three substituents R⁵, R⁶ and R⁷ represents the group -(CH₂)_{y}-O- and the other two independently of one another represent hydrogen, a hydroxy group or an amino group, |
| 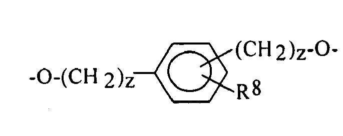 | where z is an integer of 1 to 5 and R⁸ represents hydrogen, halogen, a C₁₋₄ alkyl group, a C₁₋₄ hydroxyalkyl group, a C₂₋₄ dihydroxyalkyl group or a C₁₋₄ hydroxyalkoxy group. |
or physiologically compatible salts thereof.

2. A preparation as claimed in claim 1, **characterized in that** the substituents A and B are -NH₂ groups.

3. A preparation as claimed in claim 1 or 2, **characterized in that** R¹ is a methyl group.

4. A preparation as claimed in any of claims 1 to 3, **characterized in that** R² is hydrogen or a methyl group.

5. A preparation as claimed in any of claims 1 to 4, **characterized in that** the R³ and R⁴ represent hydrogen.

6. A preparation as claimed in any of claims 1 to 5, **characterized in that** m or n has a value of 3 or 4 and x has a value of 1 or 2.

7. A preparation as claimed in any of claims 1 to 4, **characterized in that** y or z is an integer of 1 to 3.

8. A preparation as claimed in any of claims 1 to 4, **characterized in that** one of the substituents R⁵ to R⁷ stands for the group -(CH₂)_{y}-O- and the other two stand for hydrogen.

9. A preparation as claimed in any of claims 1 to 4, **characterized in that** R⁸ is hydrogen.

10. A preparation as claimed in any of claims 1 to 4, **characterized in that** the compound corresponding to general formula (I) is 1,3-bis-(2,4-diamino-3,6-dimethylphenoxy)-propane or one of its physiologically compatible salts.

11. A preparation as claimed in any of claims 1 to 4, **characterized in that** the compound corresponding to general formula (I) is 1,3-bis-(2,4-diamino-6-methylphenoxy)-propane or one of its physiologically compatible salts.

12. A preparation as claimed in any of claims 1 to 11, **characterized in that** at least one primary intermediate is selected from p-phenylenediamine, p-toluylenediamine, 2-(2'-hydroxyethyl)-p-phenylenediamine, 2-(α,β-dihydroxyethyl)-p-phenylenediamine, N,N-bis-(2'-hydroxyethyl)-p-phenylenediamine, N,N-bis-(2'-hydroxyethyl)-amino-p-phenylenediamine, (N,N'-bis-(2'-hydroxyethyl)-N,N'-bis-(4'-aminophenyl)-1,3-diaminopropan-2-ol, bis-(2-hydroxy-5-aminophenyl)-methane, N,N'-bis-(4'-aminophenyl)-1,4-diazacycloheptane, 1,10-bis-(2',5'-diaminophenyl)-1,4,7,10-tetraoxadecane, p-aminophenol, 4-amino-3-methylphenol, 4-amino-3-fluorophenol, 4-amino-2-aminomethylphenol, 4-amino-2-((diethylamino)methyl)phenol, 2-amino-4-methylphenol, 2-amino-4-chlorophenol, 4-amino-2-(α,β-dihydroxyethyl)-phenol, 2,5-diaminopyridine, 2,4,5,6-tetraaminopyrimidine, 4-hydroxy-2,5,6-triaminopyrimidine, 2-hydroxy-4,5,6-triaminopyrimidine, 2-dimethylamino-4,5,6-triaminopyrimidine, 2,4-dihydroxy-5,6-diaminopyrimidine, 2,5,6-triaminopyrimidine, 1-(2'-hydroxy-5'-aminobenzyl)-imidazolidin-2-one and 4,5-diamino-1-(2'-hydroxyethyl)-pyrazole.

13. A preparation as claimed in any of claims 1 to 12, **characterized in that** it contains at least one other secondary intermediate selected from 2-amino-3-hydroxy-5-chloropyridine, 2,6-dihydroxy-3,4-dimethylpyridine, 2,6-bis-(2-hydroxyethylamino)-toluene, 3-amino-2,4-dichlorophenol, 3-amino-2-chloro-6-methylphenol, 5-amino-4-chloro-2-methylphenol, 5-(2'-hydroxyethyl)-amino-2-methylphenol, 5-amino-2-methylphenol, 2-amino-3-hydroxypyridine, 3-amino-2-methylamino-6-methoxypyridine, 2-methylresorcinol, 4-chlororesorcinol, 2,4-diaminophenoxyethanol, 1,3-bis-(2,4-aminophenoxy)-propane, resorcinol, m-aminophenol, 3,5-diamino-2,6-dimethoxypyridine, 1,7-, 2,7- and 1,5-dihydroxynaphthalene and 4-hydroxyindole and 6-hydroxyindole.

14. A preparation as claimed in any of claims 1 to 13, **characterized in that** it additionally contains a substantive dye.

15. A preparation as claimed in any of claims 1 to 14, **characterized in that** it additionally contains an anionic, nonionic or cationic polymer.

16. A preparation as claimed in any of claims 1 to 15, **characterized in that** it additionally contains at least one surfactant.

17. A preparation as claimed in any of claims 1 to 16, **characterized in that** it additionally contains an emulsifiable fatty components, preferably a C₁₂₋₂₂ fatty alcohol mixture.

18. A process for the oxidative coloring of keratin fibers, more particularly human hair, **characterized in that** the preparation claimed in any of claims 1 to 15 is applied to the fibers at a temperature of 15 to 40°C and, after a contact time of 5 to 45 minutes, is rinsed off again.

19. The use of the compounds corresponding to general formula (I) as claimed in any of claims 1 to 15 for the oxidative coloring of keratin fibers.

20. Compounds corresponding to formula (I) in claim 1 **characterized by** the following combinations of substituents:
| | **A=B** | **E** | **R¹** | **R²** |
|---|---|---|---|---|
| **1** | -NH₂ | -O-(CH₂)₃-O- | -CH₃ | -CH₃ |
| **2** | -NH₂ | -O-(CH₂)₃-O- | -CH₃ | -H |

## Revendications

1. Agent pour la teinture par oxydation de fibres kératiniques, en particulier de cheveux humains, contenant, dans un milieu approprié pour la teinture, comme précurseur de colorant, au moins un composant de couplage et au moins un composant de développement, **caractérisé en ce que** le composant de couplage est choisi parmi les composés de formule générale (I), 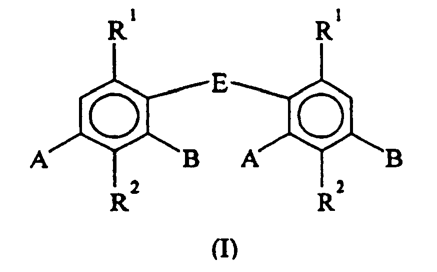 dans laquelle
- A et B représentent, indépendamment l'un de l'autre, un groupe -NH_{2,} -NHR ou -NR^{a}R^{b}, R, R^{a} et R^{b} pouvant signifier, indépendamment l'un de l'autre, un résidu C₁-C₄-alkyle, C₂-C₄-monohydroxyalkyle ou C₂-C₄-polyhydroxyalkyle,
- R¹ et R² représentent, indépendamment l'un de l'autre, halogène, un groupe C₁-C₄-alkyle, C₁-C₄-hydroxyalkyle, C₂-C₄-dihydroxyalkyle, C₁-C₄-alcoxy ou C₁-C₄-hydroxyalcoxy, à condition que R² puisse en outre représenter hydrogène,
- E représente
| | |
|---|---|
| -O-(CR³R⁴)ₘ-O- | avec : R³ et R⁴ représentant, indépendamment l'un de l'autre, hydrogène, un groupe C₁-C₄-alkyle, C₁-C₄-hydroxyalkyle, C₂-C₄-dihydroxyalkyle, C₁-C₄-alcoxy ou C₁-C₄-hydroxyalcoxy et m représentant un nombre entier de 1 à 10, |
| -O-(CₙHZ₂ₙ₋ₓ(OH)ₓ)-O- | avec : n représentant un nombre entier de 3 à 10, et x représentant un nombre de 1 à 8, à condition que n soit supérieur à x, |
| 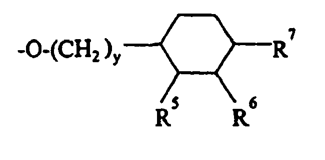 | avec : y représentant un nombre entier de 1 à 5, un des trois résidus R⁵, R⁶ et R⁷ représentant le groupement -(CH₂)y-O- et les deux autres résidus représentant, indépendamment l'un de l'autre, hydrogène, un groupe hydroxy ou un groupe amino, |
| 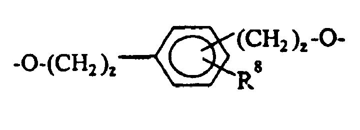 | avec : z représentant un nombre entier de 1 à 5, et R⁸ représentant hydrogène, halogène, un groupe C₁-C₄-alkyle, C₁-C₄-hydroxyalkyle, C₂-C₄-dihydroxyalkyle ou C₁-C₄-hydroxyalcoxy, |
ou leurs sels physiologiquement acceptables.

2. Agent selon la revendication 1, **caractérisé en ce que** les résidus A et B sont des groupes -NH₂.

3. Agent selon les revendications 1 ou 2, **caractérisé en ce que** R¹ représente un groupe méthyle.

4. Agent selon l'une quelconque des revendications 1 à 3, **caractérisé en ce que** R² représente hydrogène ou un groupe méthyle.

5. Agent selon l'une quelconque des revendications 1 à 4, **caractérisé en ce que** les résidus R³ et R⁴ représentent hydrogène.

6. Agent selon l'une quelconque des revendications 1 à 5, **caractérisé en ce que** m ou n représentent 3 ou 4 et x représente 1 ou 2.

7. Agent selon l'une quelconque des revendications 1 à 4, **caractérisé en ce que** y ou z représentent des nombres entiers entre 1 et 3.

8. Agent selon l'une quelconque des revendications 1 à 4, **caractérisé en ce qu'**un des résidus R⁵ à R⁷ représente le groupement -(CH₂)_{y}-O- et les deux autres résidus représentent hydrogène.

9. Agent selon l'une quelconque des revendications 1 à 4, **caractérisé en ce que** R⁸ représente hydrogène.

10. Agent selon l'une quelconque des revendications 1 à 4, **caractérisé en ce que** le composé de formule générale (I) est le 1,3-bis-(2,4-diamino-3,6-diméthylphénoxy)-propane ou un de ses sels physiologiquement acceptables.

11. Agent selon l'une quelconque des revendications 1 à 4, **caractérisé en ce que** le composé de formule générale (I) est le 1,3-bis-(2,4-diamino-6-méthylphénoxy)-propane ou un de ses sels physiologiquement acceptables.

12. Agent selon l'une quelconque des revendications 1 à 11, **caractérisé en ce qu'**au moins un composant de développement est choisi parmi la p-phénylènediamine, la p-toluylènediamine, la 2-(2'-hydroxyéthyl)-p-phénylènediamine, la 2-(α,β-dihydroxyéthyl)-p-phénylènediamine, la N,N-bis-(2'-hydroxyéthyl)-p-phénylènediamine, la N,N-bis-(2'-hydroxyéthyl)amino-p-phénylènediamine, le (N,N'-bis-(2'-hydroxyéthyl)-N,N'-bis-(4'-aminophényl)-1,3-diaminopropan-2-ol, le bis-(2-hydroxy-5-aminophényl)-méthane, le N,N'-bis-(4'-aminophényl)-1,4-diazacycloheptane, le 1,10-bis-(2',5'-diaminophényl)-1,4,7,10-tétraoxadécane, le p-aminophénol, le 4-amino-3-méthylphénol, le 4-amino-3-fluorophénol, le 4-amino-2-aminométhylphénol, le 4-amino-2-((diéthylamino)méthyl)phénol, le 2-amino-4-méthylphénol, le 2-amino-4-chlorophénol, le 4-amino-2-(α,β-dihydroxyéthyl)-phénol, la 2,5-diaminopyridine, la 2,4,5,6-tétraaminopyrimidine, la 4-hydroxy-2,5,6-triaminopyrimidine, la 2-hydroxy-4,5,6-triaminopyrimidine, la 2-diméthylamino-4,5,6-triaminopyrimidine, la 2,4-dihydroxy-5,6-diaminopyrimidine, la 2,5,6-triaminopyrimidine, la 1-(2'-hydroxy-5'-aminobenzyl)-imidazolidin-2-one et le 4,5-diamino-1-(2'-hydroxyéthyl)pyrazole.

13. Agent selon l'une quelconque des revendications 1 à 12, **caractérisé en ce qu'**il contient au moins un autre composant de couplage qui est choisi parmi la 2-amino-3-hydroxy-5-chloropyridine, la 2,6-dihydroxy-3,4-diméthylpyridine, le 2,6-bis-(2-hydroxyéthylamino)-toluène, le 3-amino-2,4-dichlorophénol, le 3-amino-2-chloro-6-méthylphénol, le 5-amino-4-chloro-2-méthylphénol, le 5-(2'-hydroxyéthyl)amino-2-méthylphénol, le 5-amino-2-méthylphénol, la 2-amino-3-hydroxypyridine, la 3-amino-2-méthylamino-6-méthoxypyridine, le 2-méthylrésorcinol, le 4-chlororésorcinol, le 2,4-diaminophénoxyéthanol, le 1,3-bis-(2,4-diaminophénoxy)propane, le résorcinol, le m-aminophénol, la 3,5-diamino-2,6-diméthoxypyridine, le 1,7-dihydroxynaphtalène, 2,7-dihydroxynaphtalène et 1,5-dihydroxynaphtalène ainsi que le 4-hydroxyindole et le 6-hydroxyindole.

14. Agent selon l'une quelconque des revendications 1 à 13, **caractérisé en ce qu'**il contient en outre un colorant montant directement sur la fibre.

15. Agent selon l'une quelconque des revendications 1 à 14, **caractérisé en ce qu'**il contient en outre un polymère anionique, non ionogène ou cationique.

16. Agent selon l'une quelconque des revendications 1 à 15, **caractérisé en ce qu'**il contient en outre au moins un agent tensioactif.

17. Agent selon l'une quelconque des revendications 1 à 16, **caractérisé en ce qu'**il contient en outre un composant gras pouvant être émulsionné, de préférence un mélange d'alcools gras en C₁₂ à C₂₂.

18. Procédé pour la teinture par oxydation de fibres kératiniques, en particulier de cheveux humains, **caractérisé en ce qu'**un agent selon l'une quelconque des revendications 1 à 15 est appliqué sur les fibres à une température entre 15 et 40°C et est à nouveau éliminé par rinçage après un temps d'action de 5 à 45 minutes.

19. Utilisation de composés de formule générale (I) selon l'une quelconque des revendications 1 à 15 pour la teinture par oxydation de fibres kératiniques.

20. Composés selon la formule (I) selon la revendication 1, qui sont **caractérisés par** les combinaisons de substituants suivantes :
| | | | | |
|---|---|---|---|---|
| | A = B | E | R¹ | R² |
| 1 | -NH₂ | -O-(CH₂)₃-O- | -CH₃ | -CH₃ |
| 2 | -NH₂ | -O-(CH₂)₃-O- | -CH₃ | -H |
